# EUROPEAN PATENT APPLICATION

(11) **EP 4 434 507 A1**
(43) Date of publication of application: **25.09.2024**
(21) Application number: 21967615.2
(22) Date of filing: 15.12.2021
(51) Int. Cl.: A61H 1/00

(54) **PHYSICAL METHOD AND APPARATUS FOR REGULATING MOLECULAR TRANSPORT IN BRAIN EXTRACELLULAR SPACE**

(71) Applicant: Peking University Third Hospital, Beijing 100191 (CN)
(72) Inventor: HAN, Hongbin, Beijing 100191 (CN)
(74) Representative: Ipside
(86) International application number: PCT/CN2021/138337
(87) International publication number: WO 2023/108478

(57) **Abstract**

A physical method for regulating molecular transport within a brain extracellular space includes: applying an external pressure to brain tissue of an animal, wherein a rhythm of applying the external pressure is related to intrinsic rhythmicity of the animal. An apparatus for regulating molecular transport within a brain extracellular space includes: a detection mechanism (10), a pressurization mechanism (30), and a control mechanism (50). The detection mechanism (10) is capable of detecting intrinsic rhythmicity of an animal. The pressurization mechanism (30) is capable of applying an external pressure to brain tissue of the animal. The control mechanism (50) is capable of control the pressurization mechanism (10) based on a detection result of the detection mechanism (30), such that a rhythm of applying the external pressure by the pressurization mechanism (30) is related to the intrinsic rhythmicity of the animal. The method and apparatus may effectively regulate molecular transport within the brain extracellular space.

## Description

### Technical Field

The present disclosure relates to a physical method and apparatus for regulating molecular transport within a brain extracellular space.

### Background of the Invention

The extracellular space (ECS) is a complex, interwoven space present between brain cells and between cells and blood vessels. Within this space, substance transport occurs in both convection and diffusion modes. A novel drug delivery method via the ECS has successfully bypassed the blood-brain barrier, which impedes most drugs from entering the brain, offering new hope for many drugs that previously failed when developed for oral and intravenous delivery routes.

The factors influencing substance transport within the brain ECS are complex; and neural excitation and post-excitation neurotransmitter release may all cause changes in the transport speed within the brain ECS (Y. Li et al., 2020; Shi et al., 2015). Various factors such as sleep (Xie et al., 2013), anesthesia (Zhao et al., 2020), neural excitation (Shi et al., 2015), and development (R. Wang et al., 2021) may positively or negatively regulate the drainage of interstitial fluid (ISF) within the brain ECS. All of these provide important theoretical foundations and technical approaches for exploring stroke treatment through the ECS pathway. For example, pain stimulation (Shi et al., 2015) and olfactory stimulation may slow down ISF drainage speed; light exposure may accelerate ISF drainage to promote the removal of ab from the brain, thereby treating Alzheimer's disease (Yue et al., 2019); and different durations of simulated microgravity (Gao et al., 2021) and different types of anesthesia (Zhao et al., 2020) may regulate ISF drainage positively or negatively.

Currently, more effective methods for regulating molecular transport within the brain extracellular space are still needed.

### Summary of the Invention

One object of the present disclosure is to provide a method for regulating molecular transport within a brain extracellular space.

Another object of the present disclosure is to provide an apparatus for regulating molecular transport within a brain extracellular space.

The present disclosure provides a physical method for regulating molecular transport within a brain extracellular space. The method includes: applying an external pressure to brain tissue of an animal, wherein a rhythm of applying the external pressure is related to intrinsic rhythmicity of the animal.

The method may effectively regulate molecular transport within the brain extracellular space.

In some exemplary embodiments, the intrinsic rhythmicity is respiratory rhythm, a heart rhythm, a brain pulsation rhythm, or a vascular pulsation rhythm.

In some exemplary embodiments, the external pressure is applied to the brain tissue of the animal from a lateral side of dura mater of the animal.

The present disclosure further provides an apparatus for regulating molecular transport within a brain extracellular space includes: a detection mechanism, a pressurization mechanism, and a control mechanism. The detection mechanism is capable of detecting intrinsic rhythmicity of an animal. The pressurization mechanism is capable of applying an external pressure to brain tissue of the animal. The control mechanism is capable of control the pressurization mechanism based on a detection result of the detection mechanism, such that a rhythm of applying the external pressure by the pressurization mechanism is related to the intrinsic rhythmicity of the animal. The apparatus may effectively regulate molecular transport within the brain extracellular space.

In some exemplary embodiments, the intrinsic rhythmicity is respiratory rhythm, a heart rhythm, a brain pulsation rhythm, or a vascular pulsation rhythm.

In some exemplary embodiments, the apparatus includes a flexible bladder, a fluid container, and a fluid charging and discharging unit. The flexible bladder is capable of applying the external pressure to the brain tissue of the animal by injecting fluid. The fluid container is capable of storing fluid. The fluid charging and discharging unit is communicated with the flexible bladder and the fluid container, and is capable of injecting fluid stored in the fluid container into the flexible bladder and also capable of discharging fluid from the flexible bladder. The control mechanism is capable of controlling the fluid charging and discharging unit.

In some exemplary embodiments, the detection mechanism is an electrocardiogram monitor, and the control mechanism is capable of extracting QRS wave and T wave from the detection result of the detection mechanism; and the control mechanism is capable of controlling the fluid charging and discharging unit to inject fluid into the flexible bladder at a start point of the QRS wave, and controlling the fluid charging and discharging unit to discharge fluid from the flexible bladder at an end point of the T wave.

In some exemplary embodiments, the fluid charging and discharging unit includes an intake tube, a gas compressor, an exhaust tube, and an exhaust valve. One end of the intake tube is communicated with the flexible bladder, and the other end of the intake tube is communicated with an outlet of the gas compressor. An inlet of the gas compressor is communicated with the fluid container. One end of the exhaust tube is communicated with the flexible bladder, and the other end of the exhaust tube is communicated with the exhaust valve. The control mechanism is capable of controlling the gas compressor and the exhaust valve.

In some exemplary embodiments, the fluid charging and discharging unit further includes an intake valve arranged between the gas compressor and the fluid container.

In some exemplary embodiments, the fluid charging and discharging unit further includes a flowmeter connected to the control mechanism. The flowmeter is arranged in the intake tube and the exhaust tube to detect a volume of fluid flowing through the intake tube and the exhaust tube . The control mechanism is capable of accumulate a total intake volume and/or a total exhaust volume based on a detection result of the flowmeter. The apparatus further includes a display unit, wherein the display unit is connected to the control mechanism, and the control mechanism is capable of controlling the display unit to display the detection result of the detection mechanism, and the total intake volume and/or the total exhaust volume.

In some exemplary embodiments, the apparatus includes a motor and an actuator plate. The control mechanism is capable of controlling the motor to operate. The actuator plate is connected to an output end of the motor, wherein the motor is capable of driving the actuator plate to move to apply the external pressure to the brain tissue of the animal.

In some exemplary embodiments, the apparatus further includes a pressure sensor. The pressure sensor is arranged in the pressurization mechanism, and is configured to detect the external force applied by the pressurization mechanism to the brain tissue. The pressure sensor is connected to the control mechanism, and the control mechanism is capable of controlling the pressurization mechanism based on a detection result of the pressurization mechanism, such that a maximum value of the external pressure applied by the pressurization mechanism to the brain tissue of the animal satisfies a predetermined value.

In some exemplary embodiments, the apparatus further includes an input mechanism. The input mechanism is connected to the control mechanism, and is capable of inputting an operating parameter and a turn-on or turn-off signal to the control mechanism. The operating parameter includes the predetermined value of the maximum value of the external pressure applied by the pressurization mechanism to the brain tissue of the animal.

### Brief description of the Drawings

The accompanying drawings are merely for schematic and illustrative description and demonstration of the present disclosure, instead of limiting the scope of the present disclosure.
FIG. 1 is a schematic structural diagram of an apparatus for regulating molecular transport within a brain extracellular space according to some exemplary embodiments of the present disclosure;
FIG. 2 is a schematic structural diagram of a pressurization mechanism of the apparatus for regulating molecular transport within a brain extracellular space according to some exemplary embodiments of the present disclosure;
FIG. 4A to FIG. 4D are NMR scanning images in effect experiments;
FIG. 5 to FIG. 8 are bar charts showing structural characteristic values of the brain ECS in the effect experiments;
FIG. 9A to FIG. 9D are two-dimensional plots reflecting a molecular diffusion coefficient in the brain ECS; and
FIG. 10Ato FIG. 10D are three-dimensional plots reflecting the molecular diffusion coefficient in the brain ECS.

Reference numerals and denotations thereof:
10-detection mechanism
30-pressurization mechanism
31-flexible bladder
32-fluid container
33-fluid charging and discharging unit
34-intake tube
332-gas compressor
333-exhaust tube
334-exhaust valve
335-flowmeter
336-intake valve
50-control mechanism
51-preamplifier
52-high-pass filter
53-pulse amplitude and frequency extraction circuit
54-microcontroller
60-display unit
70-pressure sensor
80-input mechanism
91-skull
92-dura mater
93-pia mater

### Detailed Description of Embodiments

For clearer descriptions of the technical features, objects, and the technical effects of the present disclosure, the specific embodiments of the present disclosure are hereinafter described with reference to the accompanying drawings. In the drawings, like reference numerals denote elements having the same structure or having the similar structure but the same function.

In this text, the term "exemplary" or "schematic" is used herein to mean "serving as an example, instance, or illustration," and any illustration or embodiment described herein as "exemplary" shall not be necessarily construed as preferred or advantageous over other illustrations or embodiments.

For brevity, parts relevant to the present disclosure are merely illustrated in the drawings, and these parts do not denote the actual structure of the product.

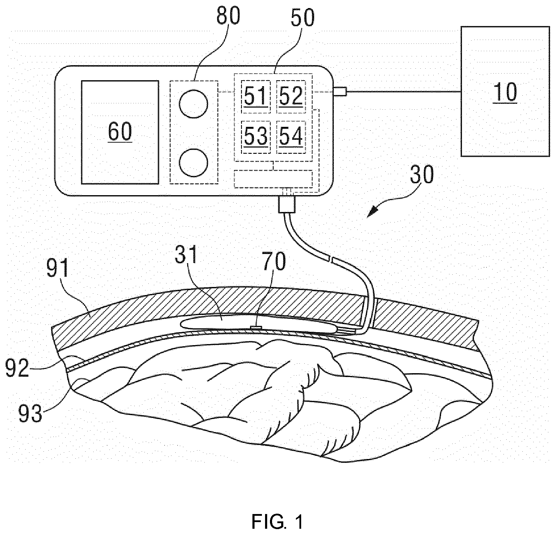

FIG. 1 is a schematic structural diagram of an apparatus for regulating molecular transport within a brain extracellular space according to some exemplary embodiments of the present disclosure. As illustrated in FIG. 1, the apparatus includes: a detection mechanism 10, a pressurization mechanism 30, and a control mechanism 50.

The detection mechanism 10 is capable of detecting intrinsic rhythmicity of an animal. As for the intrinsic rhythmicity (automaticity for short), generally, when a system acts without any external stimulation, the system has intrinsic rhythmicity. In the perspective of physiology, the intrinsic rhythmicity typically refers to the ability of a part or organ of the body to continue its excitable activity without any additional stimulation. The intrinsic rhythmicity may be, for example, respiratory rhythm, a heart rhythm, a brain pulsation rhythm, or a vascular pulsation rhythm. When the intrinsic rhythmicity is the heart rhythm, the detection mechanism 10 is, for example, an electrocardiogram monitor.

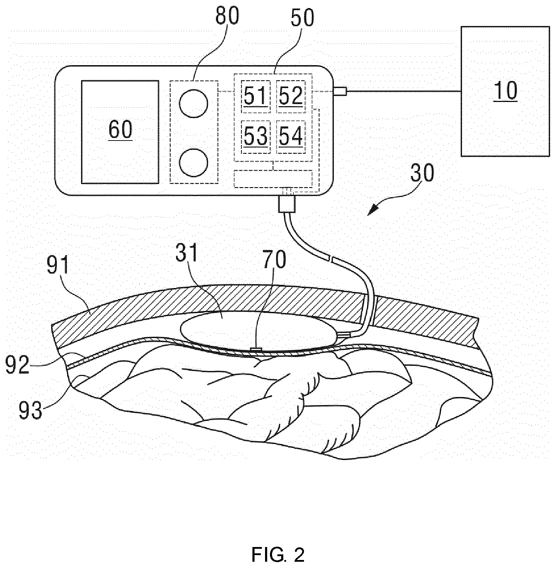

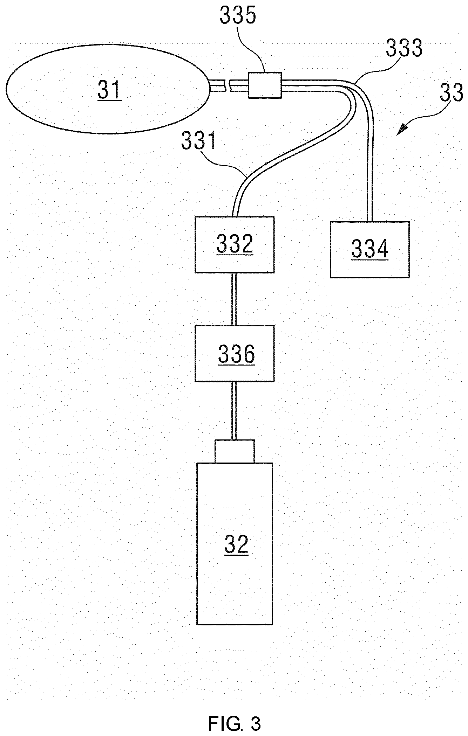

The pressurization mechanism 30 is capable of applying an external pressure to brain tissue of the animal. The external pressure refers to a force applied by a structure outside the brain tissue to the brain tissue. FIG. 3 is a schematic diagram of a specific structure of the pressurization mechanism. Specifically, as illustrated in FIG. 3, in some exemplary embodiments, the pressurization mechanism 30 includes a flexible bladder 31, a fluid container 32 configured to store fluid, and a fluid charging and discharging unit 33. The flexible bladder 31 is capable of applying the external pressure to the brain tissue of the animal by injecting fluid. As illustrated in FIG. 1 and FIG. 2, during use, the flexible bladder 31 is, for example, arranged between skull 91 and dura mater 92 of the animal. However, in other practices, the flexible bladder 31 may be, for example, arranged on a side, facing away from the skull 91, of the dura mater 92 of the animal. The flexible bladder 31 is made of, for example, a polyurethane polymer material, which has good flexibility. The flexible bladder 31 is configured with such a size that the flexible bladder 31 is positionable between the skull 91 and the dura mater 92, with a small thickness as much as possible. FIG. 1 illustrates a state of the flexible bladder 31 with fluid discharged, and FIG. 2 illustrates a state of the flexible bladder 31 with fluid injected applying a pressure to the brain tissue. The fluid may be gas, wherein the gas is, for example, helium. In some exemplary embodiments, the fluid may also be liquid. The fluid charging and discharging unit 33 is communicated with the flexible bladder 31 and the fluid container 32. The fluid charging and discharging unit 33 is capable of injecting fluid stored in the fluid container 32 into the flexible bladder 31. The fluid charging and discharging unit 32 is capable of exhausting fluid from the flexible bladder 31. In some exemplary embodiments, the fluid container 32 is, for example, a high-pressure gas storage tank.

Specifically, as illustrated in FIG. 3, in the exemplary embodiments of the present disclosure, the fluid charging and discharging unit 33 includes, for example, an intake tube 331, a gas compressor 332, an exhaust tube 333, and an exhaust valve 334. One end of the intake tube 331 is communicated with the flexible bladder 31. An outlet of the gas compressor 332 is communicated with the other end of the intake tube 331 and an inlet of the gas compressor 332 is communicated with the fluid container 32. One end of the exhaust tube 333 is communicated with the flexible bladder 31. The exhaust valve 334 is communicated with the other end of the exhaust tube 333, and the exhaust valve 334 is, for example, a solenoid valve.

The control mechanism 50 is capable of control the pressurization mechanism 10 based on a detection result of the detection mechanism 30, such that a rhythm of applying the external pressure by the pressurization mechanism 30 is related to the intrinsic rhythmicity of the animal. Specifically, in the exemplary embodiments, the controller mechanism 50 is capable of controlling the fluid charging and discharging unit 33, and further, the control mechanism 50 is capable of controlling the gas compressor 332 and the exhaust valve 334.

The apparatus is capable of applying an external pressure to brain tissue of an animal, wherein a rhythm of applying the external pressure is related to intrinsic rhythmicity of the animal.

In some exemplary embodiments, the fluid charging and discharging unit 33 further includes an intake valve 336 arranged between the gas compressor 332 and the fluid container 32.

In the exemplary embodiments, the detection mechanism 10 is, for example, an electrocardiogram monitor, and the control mechanism 50 is capable of, for example, extracting QRS wave and T wave from the detection result of the detection mechanism 10. The control mechanism 50 is capable of controlling the fluid charging and discharging unit 33 to inject fluid into the flexible bladder 31 at a start point of the QRS wave, and controlling the fluid charging and discharging unit 33 to discharge fluid from the flexible bladder 31 at an end point of the T wave. In this way, precise control is facilitated. Specifically, in the exemplary embodiments, the control mechanism 50 includes, for example, a preamplifier 51, a high-pass filter 52, a pulse amplitude and frequency extraction circuit 53, and a microcontroller 54. Electrocardiogram signals measured by the electrocardiogram monitor are sent to the preamplifier 51 and the high-pass filter 52 for removal of baseline, and then enter the pulse amplitude and frequency extraction circuit 53 and are shaped into digital signals in the pulse amplitude and frequency extraction circuit 53. Afterwards, the digital signals are fed to the microcontroller 54, and the microcontroller 54 is capable of controlling the exhaust valve 334 and the intake valve 336.

As illustrated in FIG. 3, in some exemplary embodiments, the fluid charging and discharging mechanism 33 further includes a flowmeter 335. The flowmeter 335 is arranged in the intake tube 331 and the exhaust tube 333 to detect a volume of fluid flowing through the intake tube 331 and the exhaust tube 333. The flowmeter 335 is connected to the control mechanism 50. The control mechanism 50 is capable of accumulate a total intake volume and/or a total exhaust volume based on a detection result of the flowmeter 335. In this way, it is convenient for a user to monitor the operation of the apparatus.

As illustrated in FIG. 1, in some exemplary embodiments, the apparatus further includes a display unit 60 connected to the control mechanism 50. The control mechanism 50 is capable of controlling the display unit 60 to display the detection result of the detection mechanism 10, and the total intake volume and/or the total exhaust volume. In this way, it is convenient for a user to monitor the operation of the apparatus.

As illustrated in FIG. 1, in some exemplary embodiments, the apparatus further includes a pressure sensor 70, wherein the pressure sensor 70 is arranged in the pressurization mechanism 30 and is configured to detect the external pressure applied by the pressurization mechanism 30 to the brain tissue. The pressure sensor 70 is connected to the control mechanism 50. The control mechanism 50 is capable of controlling the pressurization mechanism 30 based on a detection result of the pressurization mechanism 70, such that a maximum value of the external pressure applied by the pressurization mechanism 30 to the brain tissue of the animal satisfies a predetermined value. In this way, it is convenient to control a magnitude of the pressure.

As illustrated in FIG. 1, in some exemplary embodiments, the apparatus further includes an input mechanism 80 connected to the control mechanism 50. The input mechanism 80 is capable of inputting an operating parameter and a turn-on or turn-off signal to the control mechanism 50, wherein the operating parameter includes the predetermined value of the maximum value of the external pressure applied by the pressurization mechanism 30 to the brain tissue of the animal. The input mechanism is, for example, a key. However, in some exemplary embodiments, the input mechanism 80 may also be, for example, a knob or a touch mechanism (for example, a touch keypad or keyboard). In this way, it is convenient for a user to control the apparatus.

In some exemplary embodiments, the apparatus further includes a battery to provide power energy required for the operation of the apparatus.

In some exemplary embodiments, the pressurization mechanism may further include, for example, a motor and an actuator plate. The control mechanism is capable of controlling the motor to operate. The actuator plate is connected to an output end of the motor, wherein the motor is capable of driving the actuator plate to move to apply the external pressure to the brain tissue of the animal. However, the pressurization mechanism may also be a structure capable of applying an external pressure to the brain tissue of the animal.

Hereinafter, applying the external pressure to the brain tissue of the animal, impacts on the molecular transport within the brain extracellular space due to correlation between the rhythm of applying the external pressure and the intrinsic rhythmicity of the animal are verified or confirmed by experiments.

### Effect experiment

In this experiment, an external pressure was applied to brain tissue of rats using pulsation of the rats' own arteries to synchronize a rhythm of applying the external pressure with a pulsation rhythm of the arteries.

The rats were randomly divided into four groups according to the experimental requirements:
Control group (Group A): No arterial patching performed.
Arterial patching surgery group (Group B): Arterial patching surgery and ipsilateral contrast agent application performed.
Arterial patching surgery contralateral group (Group C): Arterial patching surgery and contralateral contrast agent application performed.
Arterial patching with gelatin sponge pad group (Group D): Arterial patching surgery, ipsilateral contrast agent application, and gelatin sponge pad application performed.

### 1. Treatment of experimental animals

Arterial patching: The rats' own arteries were placed epidurally on the right side of the brain.

Ipsilateral contrast agent: The contrast agent was present in the rat's right caudate nucleus.

Contralateral contrast agent: The contrast agent was present in the rat's left caudate nucleus.

Gelatin sponge pad application: A gelatin sponge pad was placed under the artery used for arterial patching to counteract the effects of arterial pulsation.

### 2. NMR scanning and data post-processing

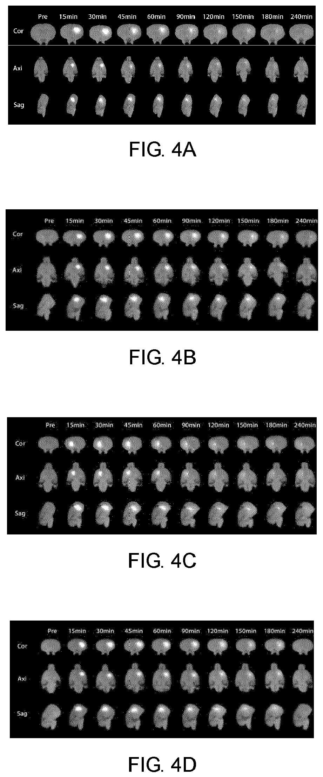

MRI scanning was performed on rats in the prone position, and images were collected until the contrast agent had fully diffused. The collected images are shown in FIGS. 4A, 4B, 4C, and 4D, corresponding to groups A, B, C, and D, respectively. In the images, Cor: coronal imaging of the rat brain; Axi: axial imaging of the rat brain; Sag: sagittal imaging; Pre: pre-contrast images; 15-240min: time points at which images were collected.

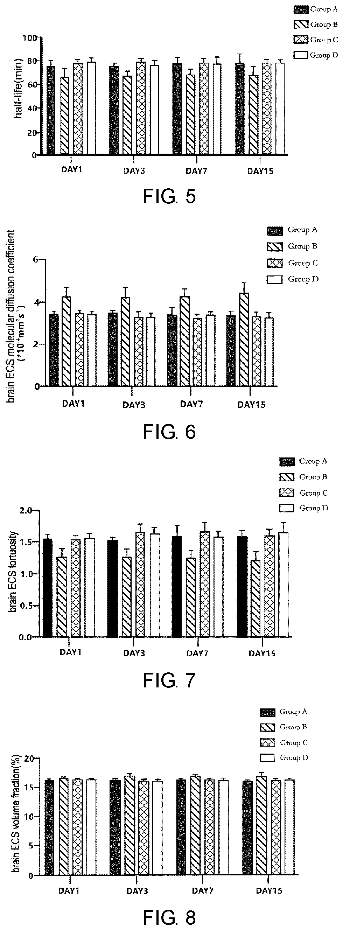

The MRI scan results were post-processed using the NanoDetect analysis software to obtain the following parameters:
contrast agent clearance half-life (T1/2), brain ECS molecular diffusion coefficient (D*), brain ECS tortuosity (λ), and brain ECS volume fraction (a). The results can be seen in Figures 5, 6, 7, and 8, respectively.

### 3. Result analysis

As shown in FIGS. 4A to 4D, the contrast agent diffuses from the caudate nucleus area to the ipsilateral superficial cortex. The rate of elimination of the bright white contrast agent spots in FIGS. 4A to 4D is relatively consistent, which is likely due to the limited resolution of the MRI images.

From FIGS. 5 to 8, it can be observed that the arterial patching surgery group (Group B) exhibits a shorter contrast agent clearance half-life (T1/2), a higher brain ECS molecular diffusion coefficient (D*), a lower brain ECS tortuosity (λ), and a higher brain ECS volume fraction (α) at 1, 3, 7, and 15 days compared to the other groups. This suggests that arterial patching can accelerate ISF drainage within the brain tissue of rats, enhance microscopic molecular diffusion, decrease tortuosity, and increase the ECS space. However, there were no statistically significant differences in the aforementioned parameters between the control group (Group A) and the arterial patching surgery contralateral group (Group C) or the arterial patching with gelatin sponge pad group (Group D).

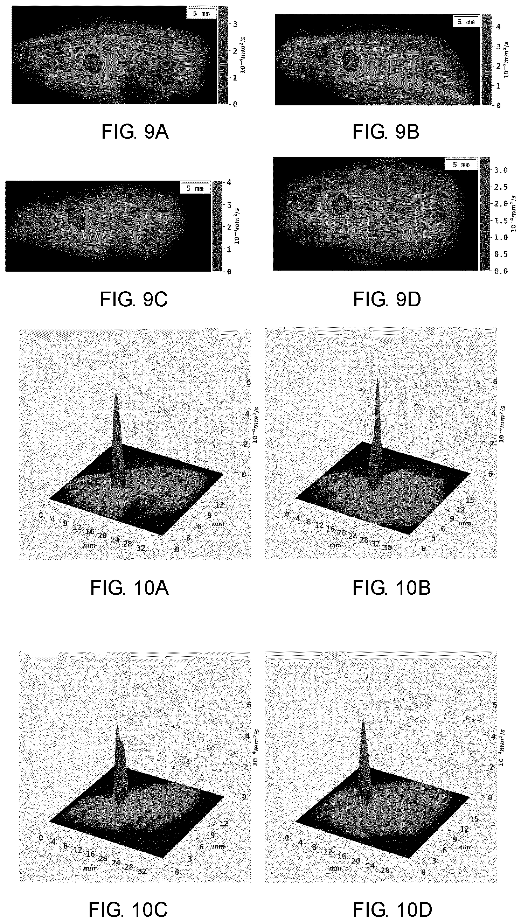

The conclusion that arterial patching can enhance the molecular diffusion coefficient within the brain ECS is also clearly demonstrated in the D-mapping visualization charts of the molecular diffusion coefficient within the brain ECS. FIG. 9A, FIG. 9B, FIG. 9C, and FIG. 9D are two-dimensional plots reflecting a molecular diffusion coefficient in the brain ECS, corresponding to Group A, Group B, Group C, and Group D, respectively. FIG. 10A, FIG. 10B, FIG. 10C, and FIG. 10D are three-dimensional plots reflecting the molecular diffusion coefficient in the brain ECS, corresponding to Group A, Group B, Group C, and Group D, respectively. The color distribution in FIGS. 9A to 9D and the heights of the 3D bars in FIGS. 10A to 10D both reflect the magnitude of the molecular diffusion coefficient within the brain ECS. According to the drawings, the arterial patching surgery group (Group B) shows the highest molecular diffusion coefficient within the brain ECS compared to the other three groups, indicating the fastest molecular diffusion capability within the brain ECS.

### 4. Experimental results

According to the experimental results described above, compared to the control group (Group A), the arterial patching surgery group (Group B) shows accelerated ISF drainage in the brain. This change can be detected from the first day after surgery and persists up to 15 days. The arterial patching surgery contralateral group (Group C) did not exhibit significant differences, indicating that arterial patching surgery can effectively improve ISF drainage from the deep to superficial layers of the brain on the same side, but not on the contralateral side. This suggests a region-specific regulatory effect.

Based on the experimental results, the brain ISF drainage in the arterial patching with gelatin sponge pad group (Group D) showed no significant difference from the control group (Group A), indicating that brain ISF drainage returns to normal when the effect of arterial pulsation is blocked. The results from FIGS. 5 to 10 show that arterial patching surgery can accelerate the rate of ISF drainage in the brain, accompanied by an increase in the ECS space, a decrease in tortuosity, and enhanced molecular diffusion capability.

This demonstrates that epidural placement of arteries can promote molecular diffusion within the brain ECS while altering the ECS structure.

The experimental approach using the rat's own arteries to apply the external pressure to the brain tissue can be understood and could be replaced by using a device, as illustrated in FIG. 1, to regulate molecular transport within the brain extracellular space. This device can apply an external pressure to the brain tissue in the same rhythm as the animal's arterial pulsation to achieve similar effects as in the arterial patching surgery group (Group B), including accelerated ISF drainage, increased ECS space, decreased tortuosity, and enhanced molecular diffusion capability. The apparatus may effectively regulate molecular transport within the brain extracellular space.

The present disclosure provides a physical method for regulating molecular transport within a brain extracellular space. The method includes: applying an external pressure to brain tissue of an animal, wherein a rhythm of applying the external pressure is related to intrinsic rhythmicity of the animal. In some exemplary embodiments, the intrinsic rhythmicity may be, for example, respiratory rhythm, a heart rhythm, a brain pulsation rhythm, or a vascular pulsation rhythm. The external pressure is, for example, applied to the brain tissue of the animal from a lateral side of dura mater of the animal. The application of the external pressure can be achieved using the arterial patching method employed in the aforementioned experiment, or by using the device shown in FIG. 1 to regulate molecular transport within the brain extracellular space of the brain.

As seen from the above effects, the method may effectively regulate molecular transport within the brain extracellular space.

A series of detailed descriptions given in this specification are merely intended to illustrate feasible embodiments of the present disclosure, instead of limiting the protection scope of the present disclosure. Any equivalent embodiments or modifications, for example, combinations, segmentations, or repetition of features, derived without departing from the spirit of the present disclosure shall fall within the protection scope of the present disclosure.

## Claims

1. A physical method for regulating molecular transport within a brain extracellular space, comprising: applying an external pressure to brain tissue of an animal, wherein a rhythm of applying the external pressure is related to intrinsic rhythmicity of the animal.

2. The method according to claim 1, wherein the intrinsic rhythmicity is respiratory rhythm, a heart rhythm, a brain pulsation rhythm, or a vascular pulsation rhythm.

3. The method according to claim 1, wherein the external pressure is applied to the brain tissue of the animal from a lateral side of dura mater of the animal.

4. An apparatus for regulating molecular transport within a brain extracellular space, comprising:
a detection mechanism (10), capable of detecting intrinsic rhythmicity of an animal;
a pressurization mechanism (30), capable of applying an external pressure to brain tissue of the animal; and
a control mechanism (50), capable of control the pressurization mechanism (30) based on a detection result of the detection mechanism (10), such that a rhythm of applying the external pressure by the pressurization mechanism (30) is related to the intrinsic rhythmicity of the animal.

5. The apparatus according to claim 4, wherein the intrinsic rhythmicity is respiratory rhythm, a heart rhythm, a brain pulsation rhythm, or a vascular pulsation rhythm.

6. The apparatus according to claim 4, wherein the pressurization mechanism (30) comprises:
a flexible bladder (31), capable of applying the external pressure to the brain tissue of the animal by injecting fluid;
a fluid container (32), capable of storing fluid; and
a fluid charging and discharging unit (33), connected to the flexible bladder (31) and the fluid container (32), wherein the fluid charging and discharging unit (33) is capable of injecting the fluid stored in the fluid container (32) into the flexible bladder (31) and is further capable of discharging the fluid in the flexible fluid (31), and the control mechanism (20) is capable of controlling the fluid charging and discharging unit (33).

7. The apparatus according to claim 6, wherein the detection mechanism (10) is an electrocardiogram monitor, and the control mechanism (50) is capable of extracting QRS wave and T wave from the detection result of the detection mechanism (10); and the control mechanism (50) is capable of controlling the fluid charging and discharging unit (33) to inject fluid into the flexible bladder (31) at a start point of the QRS wave, and controlling the fluid charging and discharging unit (33) to discharge fluid from the flexible bladder (31) at an end point of the T wave.

8. The apparatus according to claim 6, wherein the fluid charging and discharging unit (33) comprises:
an intake tube (331), one end of the intake tube (331) being communicated with the flexible bladder (31);
a gas compressor (332), an outlet of the gas compressor (332) being communicated with the other end of the intake tube (331) and an inlet of the gas compressor (332) being communicated with the fluid container (32);
an exhaust tube (333), one end of the exhaust tube (333) being in communication with the flexible bladder (31); and
an exhaust valve (334), communicated with the other end of the exhaust tube (333), wherein the control mechanism (50) is capable of controlling the gas compressor (332) and the exhaust valve (334).

9. The apparatus according to claim 8, wherein
the fluid charging and discharging unit (33) further comprises a flowmeter (335), wherein the flowmeter (335) is arranged in the intake tube (331) and the exhaust tube (333) to detect a volume of fluid flowing through the intake tube (331) and the exhaust tube (333), the flowmeter (335) is connected to the control mechanism (50), and the control mechanism (50) is capable of accumulate a total intake volume and/or a total exhaust volume based on a detection result of the flowmeter (335); and
the apparatus further comprises a display unit (60), wherein the display unit (60) is connected to the control mechanism (50), and the control mechanism (50) is capable of controlling the display unit (60) to display the detection result of the detection mechanism (10), and the total intake volume and/or the total exhaust volume.

10. The apparatus according to claim 4, wherein the pressurization mechanism (30) comprises:
a motor, wherein the control mechanism (50) is capable of controlling the motor to operate; and
an actuator plate, connected to an output end of the motor, wherein the motor is capable of driving the actuator plate to move to apply the external pressure to the brain tissue of the animal.

11. The apparatus according to claim 4, further comprising: a pressure sensor (70), wherein the pressure sensor (70) is arranged in the pressurization mechanism (30) and is configured to detect the external pressure applied by the pressurization mechanism (30) to the brain tissue, the pressure sensor (70) is connected to the control mechanism (50), and the control mechanism (50) is capable of controlling the pressurization mechanism (30) based on a detection result of the pressure sensor (70), such that a maximum value of the external pressure applied by the pressurization mechanism (30) to the brain tissue of the animal satisfies a predetermined value.

12. The apparatus according to claim 11, further comprising: an input mechanism (80) connected to the control mechanism (50), wherein the input mechanism (80) is capable of inputting an operating parameter and a turn-on or turn-off signal to the control mechanism (50), the operating parameter comprising the predetermined value of the maximum value of the external pressure applied by the pressurization mechanism (30) to the brain tissue of the animal.
